# EUROPÄISCHE PATENTSCHRIFT

(11) **EP 0 483 396 B1**
(45) Veröffentlichungstag und Bekanntmachung des Hinweises auf die Patenterteilung: **22.12.1999**
(21) Anmeldenummer: 90120740.7
(22) Anmeldetag: 29.10.1990
(51) Int. Cl.: A61B 17/22, G10K 9/12

(54) **Akusticher Druckimpulsgenerator**
Acoustic pressure impulse generator
Générateur d'impulsions de pression acoustique

(43) Veröffentlichungstag der Anmeldung: 06.05.1992
(73) Patentinhaber: SIEMENS AKTIENGESELLSCHAFT, 80333 München (DE)
(72) Erfinder: Buchholtz, Gerhard, Dipl.-Ing., W-8520 Erlangen (DE); Mahler, Matthias, Dipl.-Ing., W-8520 Erlangen (DE); Rattner, Manfred, Ing., W-8521 Grossenseebach (DE)

(56) Entgegenhaltungen:
- EP-A- 0 265 741
- EP-A- 0 301 360
- DE-A- 3 312 014
- DE-A- 3 727 692

## Beschreibung

Die Erfindung betrifft einen Druckimpulsgenerator mit einer Druckimpulsquelle, welche zur Erzeugung von akustischen Druckimpulsen in einem akustischen Ausbreitungsmedium dient und eine stoßartig antreibbare Membran aufweist.

Derartige Druckimpulsgeneratoren können für die unterschiedlichsten Zwecke verwendet werden, z.B. in der Medizin, um im Körper eines Patienten befindliche Konkremente nichtinvasiv zu zertrümmern oder pathologische Gewebeveränderungen ebenfalls nichtinvasiv zu behandeln, wobei im ersten Fall positive (Überdruck) und im zweiten Fall vorzugsweise negative (Unterdruck) Druckimpulse verwendet werden. Außerdem können derartige Druckimpulsgeneratoren in der Werkstoffprüfung eingesetzt werden, um Materialproben mit Druckimpulsen zu beaufschlagen. Der Druckimpulsgenerator wird stets in geeigneter Weise mit dem jeweils zu beschallenden Objekt akustisch gekoppelt, so daß die in dem akustischen Ausbreitungsmedium erzeugten Druckimpulse in das Objekt eingeleitet werden können. Der Druckimpulsgenerator und das zu beschallende Objekt müssen dabei relativ zueinander so ausgerichtet sein, daß der zu beschallende Bereich des Objektes sich im Ausbreitungsweg der Druckimpulse befindet. Falls der Druckimpulsgenerator fokussierte Stoßwellen abgibt, muß außerdem sichergestellt sein, daß sich der zu beschallende Bereich des Objektes in dem Fokusbereich der Druckimpulse befindet.

Ein Druckimpulsgenerator der eingangs genannten Art ist in der US-PS 4 674 505 beschrieben. Es handelt sich hierbei um eine positive Druckimpulse erzeugenden sogenannten elektromagnetischen Stoßwellengenerator, dessen Wirkung darauf beruht, daß eine Spulenanordnung bei Beaufschlagung mit einem Hochspannungsimpuls äußerst rasch ein Magnetfeld aufbaut, welches in eine der Spulenanordnung gegenüberliegende Membran einen Strom induziert, der dem durch die Spulenanordnung fließenden Strom entgegengesetzt ist und von einem dem zur Spulenanordnung gehörigen Magnetfeld entgegengesetzten Magnetfeld begleitet wird. Infolge der hierbei auftretenden Abstoßungskräfte wird die Membran schlagartig von der Spulenanordnung wegbewegt. Dabei wird ein Druckimpuls in das akustische Ausbreitungsmedium eingeleitet, der sich auf seinem Ausbreitungsweg allmählich zur Stoßwelle aufsteilt.

Derartige Stoßwellengeneratoren besitzen einen vergleichsweise geringen Wirkungsgrad, so daß ein hoher Anteil der zugeführten elektrischen Energie in Wärme umgewandelt wird, mit der Folge, daß sich die Druckimpulsquelle allmählich aufheizt. Da erhöhte Betriebstemperaturen der Druckimpulsquelle zu deren vorzeitigem Ausfall, insbesondere wegen Versagens der hohen mechanischen Beanspruchungen unterliegenden Membran, führen kann, ist im Falle eines aus der EP-A-0 265 741 bekannten Druckimpulsgenerators vorgesehen, daß das akustische Ausbreitungsmedium während des Betriebs der Druckimpulsquelle in einem Kreislauf durch einen Kühler geleitet wird. Auf diese Weise ist es zwar grundsätzlich möglich, die von der Druckimpulsquelle an das akustische Ausbreitungsmedium abgegebene Wärme abzuführen, jedoch hat sich im Falle des bekannten Druckimpulsgenerators gezeigt, daß die Kühlwirkung nicht unter allen Umständen ausreichend ist. In dem bekannten Druckimpulsgenerator können sich nämlich "Hitzenester" bilden, aus denen eine ausreichende Wärmeabfuhr nicht gewährleistet ist. Außerdem tritt bei Druckimpulsgeneratoren der eingangs genannten Art das Problem auf, daß die Membran nach Erzeugung eines Druckimpulses in ihre Ausgangslage zurückgeführt werden muß. Nur so ist gewährleistet, daß die Membran vor Erzeugung einer Stoßwelle jeweils eine definierte Ausgangslage einnimmt, in der sie an die Spulenanordnung angepreßt ist, was Voraussetzung dafür ist, daß aufeinanderfolgend erzeugte Stoßwellen hinsichtlich ihrer akustischen Kennwerte ausreichend genau übereinstimmen. Bei einem aus der EP-A-0 188 750 bekannten Druckimpulsgenerator wird die Rückführung der Membran in ihrer Ausgangslage dadurch bewerkstelligt, daß die von dem akustischen Ausbreitungsmedium abgewandte Seite der Membran mit Unterdruck beaufschlagt wird. Auf diese Weise ist zwar eine zuverlässige Rückführung der Membran in ihrer Ausgangslage gewährleistet, jedoch muß ein ganz erheblicher konstruktiver Aufwand getrieben und eine Unterdruckquelle bereitgestellt werden.

Der Erfindung liegt die Aufgabe zugrunde, einen Druckimpulsgenerator der eingangs genannten Art so auszubilden, daß auf kostengünstige und konstruktiv einfache Weise einerseits ein vorzeitiger Ausfall des Druckimpulsgenerators infolge von ungenügender Kühlung vermieden und andererseits die Erzeugung von hinsichtlich ihrer akustischen Kennwerte im wesentlichen übereinstimmenden aufeinanderfolgenden Druckimpulsen gewährleistet ist.

Nach der Erfindung wird diese Aufgabe gelöst durch einen Druckimpulsgenerator mit einer Druckimpulsquelle, welche zur Erzeugung von akustischen Druckimpulsen in einem akustischen Ausbreitungsmedium eine stoßartig antreibbare Membran aufweist, und mit einer der Membran gegenüberliegenden Wand welcher einen zwischen der Wand und der Membran befindlichen, ein flüssiges akustisches Ausbreitungsmedium enthaltenden ersten Raum flüssigkeitsdicht von einem auf der anderen Seite der Wand befindlichen, ein akustisches Ausbreitungsmedium enthaltenden zweiten Raum trennt, wobei das in dem ersten Raum enthaltene akustische Ausbreitungsmedium unter einem zur Rückführung der Membran in ihre Ausgangslage gegenüber dem Umgebungsdruck erhöhten statistischen Druck steht und durch eine Kühleinrichtung strömt. Infolge des Umstandes, daß sich das durch eine Kühleinrichtung strömende akustische Ausbreitungsmedium in dem durch die Membran und die Wand begrenzten ersten Raum befindet, sind für das akustische Ausbreitungsmedium gute Strömungsverhältnisse vorhanden, so daß sich keine Hitzenester bilden können und eine gute Kühlwirkung somit sichergestellt ist. Zugleich wird die Membran durch das gegenüber dem Umgebungsdruck unter einem erhöhten Druck stehende akustische Ausbreitungsmedium nach Abgabe eines Druckimpulses jeweils in ihre Ausgangslage zurückgeführt, so daß in vorteilhafter Weise die zur Beaufschlagung der Rückseite der Membran mit Unterdruck erforderlichen Maßnahmen entfallen können. Dabei genügt je nach Steifigkeit der Membran übrigens unter Umständen sogar bereits ein relativ geringer Überdruck in der Größenordnung von weniger als 1 bar um eine sichere Rückführung der Membran zu gewährleisten, so daß der zur Aufrechterhaltung der Strömung des akustischen Ausbreitungsmediums ohnehin erforderliche Druck in der Regel auch ausreicht, um die Membran in ihre Ausgangslage zurückzuführen. Der erfindungsgemäße Druckimpulsgenerator weist den zusätzlichen Vorteil auf, daß infolge des in dem ersten Raum herrschenden erhöhten Druckes, die Gefahr des Auftretens von schädlichen Kavitationserscheinungen deutlich vermindert ist, und daß etwaige dennoch entstehende Kavitationsblasen infolge der Strömung des akustischen Ausbreitungsmediums rasch aus dem ersten Raum entfernt werden. Es versteht sich übrigens, daß die Wand vorzugsweise aus einem Material gebildet ist, das eine geringe akustische Dämpfung aufweist und hinsichtlich der akustischen Impedanz an die in den ersten und zweiten Raum befindlichen akustischen Ausbreitungsmedien angepaßt ist.

Aus der DE-A-33 12 014 ist zwar ein Druckimpulsgenerator bekannt, dessen Membran durch ein unter Druck stehendes Ausbreitungsmedium in ihre Ausgangslage zurückgeführt wird, jedoch ist hier weder vorgesehen, daß das akustische Ausbreitungsmedium durch eine Kühleinrichtung strömt, noch ist eine aus einem Stoßwellen leitenden Werkstoff gebildete Wand vorgesehen, die zur Sicherstellung für eine gute Kühlwirkung notwendiger und günstiger Strömungsverhältnisse dient.

Aus der DE 37 27 692 ist es übrigens bekannt, im Falle eines Druckimpulsgenerators eine zwischen dessen Membran und einer dieser gegenüberliegenden Wand befindliche Linsenflüssigkeit in einem Kreislauf strömen zu lassen, der unter Umständen eine Kühleinrichtung enthalten kann.

Vorteilhafte Varianten der Erfindung sehen vor, daß der statische Druck des in dem ersten Raum enthaltenen akustischen Ausbreitungsmediums bzw. die pro Zeiteinheit durch die Kühleinrichtung strömende Menge des akustischen Ausbreitungsmediums einstellbar sind. Es besteht somit die Möglichkeit, die auf die Membran wirkende Rückstellkraft bzw. die Kühlwirkung den jeweiligen Betriebsbedingungen anzupassen.

Eine bevorzugte Variante der Erfindung sieht vor, daß das akustische Ausbreitungsmedium in einem geschlossenen Kreislauf durch die Kühleinrichtung strömt. Es ist dann auch leicht möglich, das akustische Ausbreitungsmedium zu entgasen und mit beispielsweise korrosionshemmenden Zusätzen zu versehen.

Eine besonders gute Kühlwirkung ergibt sich, wenn das akustische Ausbreitungsmedium in dem ersten Raum eine wenigstens im wesentlichen parallel zur Oberfläche der Membran gerichtete Strömungsrichtung aufweist.

Eine besonders bevorzugte Ausführungsform der Erfindung sieht vor, daß die den ersten von dem zweiten Raum trennende Wand als akustische Linse ausgebildet ist. Dies ist insbesondere dann von Vorteil, wenn die von der Membran ausgehenden Druckimpulse der Fokussierung bedürfen und somit ohnehin eine akustische Linse erforderlich ist.

Eine zweckmäßige Ausführungsform der Erfindung sieht vor, daß der zweite Raum durch eine flexible Koppelmembran abgeschlossen ist und mit einem Ausgleichsbehälter in Verbindung steht. Auf diese Weise ist es leicht möglich, die flexible Koppelmembran, die zur akustischen Ankoppelung des Druckimpulsgenerators an ein zu beschallendes Objekt dient, in der für die jeweils gewünschte Ausrichtung des Druckimpulsgenerators relativ zu dem zu beschallenden Objekt erforderlichen Weise zu verformen. Infolge des Umstandes, daß der erste und der zweite Raum durch die Wand bzw. durch die akustische Linse voneinander getrennt sind und nur das in dem ersten Raum befindliche akustische Ausbreitungsmedium durch die Kühleinrichtung strömt, besteht in vorteilhafter Weise die Möglichkeit, die Temperatur des in dem zweiten Raum befindlichen akustischen Ausbreitungsmediums an die Bedürfnisse des zu beschallenden Objektes, beispielsweise einen Patienten, anzupassen, ohne daß dies nachteilige Einflüsse auf die zu erzielende Kühlwirkung haben kann, die wesentlich von der normalerweise geringeren Temperatur des in dem ersten Raum befindlichen akustischen Ausbreitungsmediums abhängt.

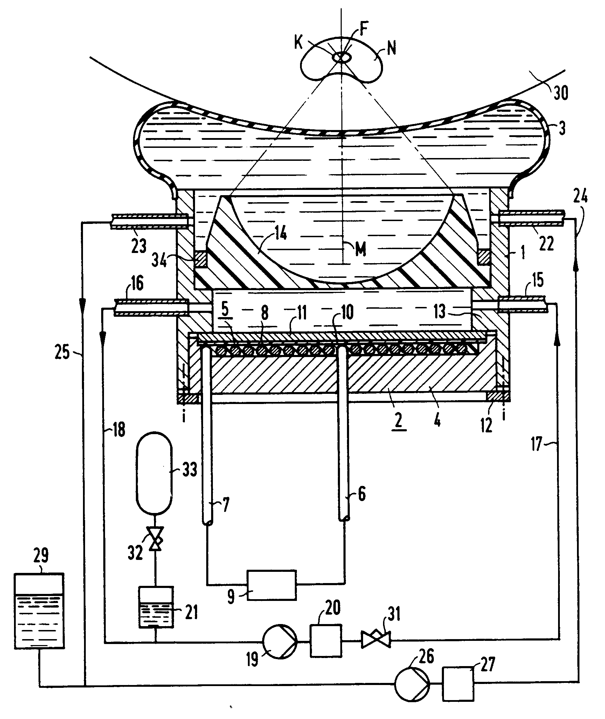

Ein Ausführungsbeispiel der Erfindung ist in der einzigen Figur der beigefügten Zeichnung dargestellt, die einen erfindungsgemäß ausgebildeten Stoßwellengenerator in schematische Darstellung im Längsschnitt zeigt.

Bei dem in der Figur dargestellten erfindungsgemäßen Druckimpulsgenerator handelt es sich um einen zur Zertrümmerung von Konkrementen im Körper eines Lebewesens dienenden Stoßwellengenerator, der ein rohrförmiges Gehäuse 1 aufweist, das an seinem einen Ende durch eine insgesamt mit 2 bezeichnete Stoßwellenquelle und an seinem anderen Ende durch eine flexible Koppelmembran 3 verschlossen ist.

Die Stoßwellenquelle 2 weist eine auf einer ebenen Auflagefläche eines Spulenträgers 4 angeordnete Spule 5 auf. Diese besitzt die Anschlüsse 6 und 7, wobei die die Anschlüsse 6 und 7 verbindenden Windungen der Spule 5, eine der Windungen ist mit dem Bezugszeichen 8 versehen, spiralförmig verlaufen. Der Spulenträger 4 ist aus einem elektrisch isolierenden Werkstoff, z.B. Aluminiumoxidkeramik, gebildet. Der Raum zwischen den Windungen 8 der Spule 5 ist mit einem elektrisch isolierenden Gießharz ausgefüllt. Die Anschlüsse 6 und 7 der Spule 5 sind mit einem elektrischen Hochspannungs-Impulsgenerator 9 verbunden.

Unter Zwischenfügung einer Isolierfolie 10 ist der von dem Spulenträger 4 abgewandten Seite der Spule 5 gegenüberliegend eine kreisscheibenförmige, ebene Membran 11 angeordnet, die aus einem elektrisch leitenden Material, beispielsweise Kupfer, besteht. Die Membran 11, die Isolierfolie 10 und die Spule 5 sind mit dem Spulenträger 4 mittels eines an diesem angebrachten Zentrierrandes zu einer Einheit zusammengefaßt. Diese Einheit ist mittels eines an dem Spulenträger 4 anliegenden Ringes 12 und mehrerer Schrauben, es sind lediglich die Mittellinien zweier Schrauben strichpunktiert angedeutet, gegen einen in der Bohrung des Gehäuses 1 vorgesehenen Absatz 13 gepreßt. Dabei liegt die Membran 11, eventuell unter Zwischenfügung geeigneter, nicht dargestellter Dichtmittel, flüssigkeitsdicht an dem Absatz 13 an. An der von der Membran 11 abgewandten Seite des Absatzes 13 liegt eine plan-konkave akustische Sammellinse 14, die beispielsweise aus Polystyrol besteht, mit ihrer planen Seite flüssigkeitsdicht an. Die Sammellinse 14 ist mittels eines schematisch angedeuteten, in die Bohrung des Gehäuses 1 eingesetzten Halteringes 34 axial fixiert. In dem zwischen der Sammellinse 14 und der Membran 11 begrenzten ersten Raum befindet sich eine Flüssigkeit, beispielsweise Wasser. Der von der Membran und der Sammellinse 14 begrenzte erste Raum weist einen Zulauf 15 und einen Ablauf 16 auf, an die eine Zulaufleitung 17 und eine Ablaufleitung 18 angeschlossen sind, so daß mittels einer Pumpe 19 das Wasser umgewälzt werden kann, das dabei durch ein Kühlaggregat 20 geleitet wird. Außerdem ist an die Ablaufleitung 18 ein Blasenabscheider 21 angeschlossen, in dem sich etwaige Gasblasen sammeln können. Der Pumpe 19 und dem Kühlaggregat 20 ist ein geeignetes Regelventil 31 nachgeschaltet, das es gestattet, die pro Zeiteinheit durch den ersten Raum und damit durch das Kühlaggregat 20 fließenden Wassermenge einzustellen. Der Blasenabscheider 21 ist unter Zwischenschaltung eines geeigneten Regelventils 32 an einen Druckluftspeicher 33 angeschlossen, so daß die Möglichkeit besteht, mittels des Regelventils 32 den in dem ersten Raum herrschenden statischen Druck einzustellen.

In einem zwischen der Sammellinse 14 und der Koppelmembran 3 befindlichen zweiten Raum, der von dem ersten Raum mittels der Sammellinse 14 flüssigkeitsdicht getrennt ist, ist ebenfalls eine Flüssigkeit, beispielsweise Wasser, enthalten. Auch der zweite Raum weist einen Zulauf 22 und einen Ablauf 23 auf, an die eine Zulaufleitung 24 und eine Ablaufleitung 25 angeschlossen sind, so daß mittels einer Pumpe 26 das Wasser durch ein Heizaggregat 27 geleitet wird. Dieses dient dazu, die Temperatur des Wassers in dem zweiten Raum etwa auf die Körpertemperatur des zu behandelnden Lebewesens 30 anzuheben. An die Ablaufleitung 25 ist ein Ausgleichsbehälter 29 angeschlossen, der dazu dient, die beim Anpressen des Stoßwellengenerators an den Körper des Lebewesens 30 aus dem zweiten Raum verdrängte Wassermenge aufzunehmen. Der Ausgleichsbehälter 29 dient zugleich als Blasenabscheider.

Mittels des beschriebenen Stoßwellengenerators werden Stoßwellen in an sich bekannter Weise erzeugt, indem die Spule 5 mittels des Hochspannungs-Impulsgenerators 9 mit einem Hochspannungsimpuls beaufschlagt wird. Die Spule 5 baut daraufhin äußerst rasch ein Magnetfeld auf, welches in die Membran 11 einen Strom induziert, der dem durch die Spule 5 fließenden Strom entgegengesetzt ist. Dieser Strom ist von einem Magnetfeld begleitet, das dem zu dem durch die Spule 5 fließenden Strom gehörigen Magnetfeld entgegengesetzt ist. Infolge der hierbei auftretenden Abstoßungskräfte wird die Membran 11 schlagartig von der Spule 5 wegbewegt, wodurch in das als akustisches Ausbreitungsmedium an die Membran 11 angrenzende, in dem ersten Raum befindliche Wasser ein zunächst ebener Druckimpuls eingeleitet wird. Dieser wird mittels der Sammellinse 14 in der in der Figur strichpunktiert angedeuteten Weise auf eine Fokuszone F fokussiert, die auf der Mittelachse M des Stoßwellengenerators liegt. Der fokussierte Druckimpuls breitet sich dann in dem in dem zweiten Raum als akustisches Ausbreitungsmedium enthaltenen Wasser aus. Wird der Stoßwellengenerator mittels des Sackes 3 unter Zuhilfenahme einer an sich bekannten Ortungsvorrichtung, z.B. einer Röntgen-Ortungsvorrichtung, in einer solchen Position an den Körper des zu behandelnden Lebewesens 30 angepreßt, daß sich ein zu zertrümmerndes Konkrement K, beispielsweise der Stein einer Niere N, in der Fokuszone F befindet, kann das Konkrement K durch eine Serie von Druckimpulsen in Fragmente zertrümmert werden, die so klein sind, daß sie auf natürlichem Wege ausgeschieden werden können. Übrigens steilen sich die von der Membran 11 ausgehenden Druckimpulse auf ihrem Weg durch das im ersten und im zweiten Raum befindliche Wasser sowie das Körpergewebe des Lebewesens 30 allmählich zu sogenannten Stoßwellen auf, bei denen es sich um Druckimpulse mit sehr steiler Anstiegsfront handelt.

Infolge des Umstandes, daß das in dem ersten Raum befindliche Wasser in der beschriebenen Weise durch das Kühlaggregat 20 zirkuliert ist sichergestellt, daß die beim Betrieb des Druckimpulsgenerators anfallende Verlustwärme problemlos abgeführt werden kann. Dabei wirkt sich auf die erzielbare Kühlwirkung positiv aus, daß durch die gegenüberliegende Anordnung des Zulaufs 17 und des Ablaufs 16 eine zu der Oberfläche der Membran 11 in etwa parallel verlaufende Strömungsrichtung vorliegt, was dem Entstehen von Hitzenestern entgegenwirkt. Weiter ist der Erzielung einer hohen Kühlwirkung förderlich, daß nur das unmittelbar im Bereich der Membran befindliche Wasser in den Kühlkreislauf einbezogen ist, was dadurch erreicht wird, daß die akustische Sammellinse 14 als Trennwand zwischen dem der Membran benachbarten ersten Raum und dem zwischen der Sammellinse 14 und der Koppelmembran 3 befindlichen zweiten Raum wirkt. Die Kühlwirkung läßt sich den jeweiligen Bedürfnissen durch Betätigen des Regelventils 31 anpassen, wobei die Kühlwirkung um so größer ist, je weiter das Regelventil 31 geöffnet ist. Außerdem kann vorgesehen sein, daß die Kühlleistung des Kühlaggregats 20 in an sich bekannter, nicht dargestellter Weise einstellbar ist. Das in dem ersten Raum befindliche Wasser steht unter einem statischen Überdruck von beispielsweise etwa 1 bar gegenüber dem Umgebungsdruck. Hierdurch wird die Membran 11, deren Dicke in der Figur übertrieben dargestellt ist, nach Erzeugung eines Druckimpulses bzw. einer Stoßwelle jeweils in ihre Ausgangslage zurückgeführt, in der sie unter Zwischenfügung der Isolierfolie 10 satt an der Oberfläche der Spule 5 anliegt. Hierdurch ist gewährleistet, daß aufeinanderfolgend erzeugte Stoßwellen jeweils die gleichen akustischen Kenngrößen aufweisen. Der statische Druck des in dem ersten Raum befindlichen Wassers läßt sich durch Betätigen des Regelventils 32 den jeweiligen Bedürfnissen anpassen. Infolge des Umstandes, daß das durch das Kühlaggregat 20 zirkulierende Wasser des ersten Raumes, das bei Eintritt in den ersten Raum beispielsweise eine unterhalb der Raumtemperatur liegende Temperatur aufweist, von dem unter Zwischenfügung der Koppelmembran 3 an den Körper des Lebewesens 30 angrenzenden Wasser des zweiten Raumes getrennt ist, kann letzteres mittels des Heizaggregates 27 auf eine für das zu behandelnde Lebewesen 30 angenehme Temperatur, z.B. Körpertemperatur, gebracht werden, ohne daß dies die im Bereich der Druckimpulsquelle 2 erzielbare Kühlwirkung nennenswert mindert. In nicht dargestellter Weise kann übrigens vorgesehen sein, daß die Kühlleistung des Kühlaggregates 20 und/oder die pro Zeiteinheit durch das Regelventil 31 strömende Wassermenge derart geregelt sind/ist, daß das Wasser mit einer vorwählbaren konstanten Temperatur in den ersten Raum einströmt. Weiter kann in ebenfalls nicht dargestellter Weise der in dem ersten Raum herrschende statische Druck in Abhängigkeit von der Amplitude der erzeugten Druckimpulse, die mit zunehmender Amplitude der der Spule 5 zugeführten Hochspannungsimpulse steigt, derart geregelt sein, daß mit zunehmender Amplitude der Druckimpulse der statische Druck steigt.

Im Falle des beschriebenen Ausführungsbeispiels ist in dem ersten und dem zweiten Raum das gleiche akustische Ausbreitungsmedium, nämlich Wasser, vorhanden. Dies muß nicht notwendigerweise der Fall sein. Vielmehr können in den beiden Räumen auch unterschiedliche akustische Ausbreitungsmedien enthalten sein.

Obwohl die Erfindung ausschließlich am Beispiel eines medizinischen Zwecken dienenden Stoßwellengenerators beschrieben ist, kann sie auch bei anderen Druckimpulsgeneratoren Verwendung finden.

## Patentansprüche

1. Druckimpulsgenerator mit einer Druckimpulsquelle (2), welche zur Erzeugung von akustischen Druckimpulsen in einem akustischen Ausbreitungsmedium eine stoßartig antreibbare Membran (11) aufweist, und mit einer der Membran (11) gegenüberliegenden Wand (14), welche einen zwischen der Wand (14) und der Membran (11) befindlichen, ein flüssiges akustisches Ausbreitungsmedium enthaltenden ersten Raum flüssigkeitsdicht von einem auf der anderen Seite der Wand (14) befindlichen, ein akustisches Ausbreitungsmedium enthaltenden zweiten Raum trennt, wobei das in dem ersten Raum enthaltene akustische Ausbreitungsmedium unter einem zur Rückführung der Membran (11) in ihre Ausgangslage gegenüber dem Umgebungsdruck erhöhten statischen Druck steht und durch eine Kühleinrichtung (20) strömt.

2. Druckimpulsgenerator nach Anspruch 1**, dadurch gekennzeichnet,** daß der statische Druck des in dem ersten Raum enthaltenen akustischen Ausbreitungsmediums einstellbar ist.

3. Druckimpulsgenerator nach Anspruch 1 oder 2, **dadurch gekennzeichnet,** daß die pro Zeiteinheit durch die Kühleinrichtung (20) strömende Menge des akustischen Ausbreitungsmediums einstellbar ist.

4. Druckimpulsgenerator nach einem der Ansprüche 1 bis 3, **dadurch gekennzeichnet,** daß das akustische Ausbreitungsmedium in einem geschlossenen Kreislauf durch die Kühleinrichtung (20) strömt.

5. Druckimpulsgenerator nach einem der Ansprüche 1 bis 4, **dadurch gekennzeichnet,** daß das akustische Ausbreitungsmedium in dem ersten Raum eine wenigstens im wesentlichen parallel zur Oberfläche der Membran (11) gerichtete Strömungsrichtung aufweist.

6. Druckimpulsgenerator nach einem der Ansprüche 1 bis 5, **dadurch gekennzeichnet,** daß die den ersten von vem zweiten Raum trennende Wand (14) als akustische Linse ausgebildet ist.

7. Druckimpulsgenerator nach einem der Ansprüche 1 bis 6, **dadurch gekennzeichnet,** daß der zweite Raum durch eine flexible Koppelmembran (3) abgeschlossen ist und mit einem Ausgleichsbehälter (29) in Verbindung steht.

## Claims

1. Pressure pulse generator comprising a pressure pulse source (2) which has a diaphragm (11) which can be driven impulsively in order to generate acoustic pressure pulses in an acoustic propagation medium, and comprising a wall (14) which is situated opposite the diaphragm (11) and separates a first space, which is situated between the wall (14) and diaphragm (11) and contains a liquid acoustic propagation medium, from a second space, which is located on the other side of the wall (14) and contains an acoustic propagation medium, the acoustic propagation medium contained in the first space being under a static pressure which is increased with respect to the ambient pressure in order to return the diaphragm (11) into its initial position, and flowing through a cooling device (20).

2. Pressure pulse generator according to Claim 1, characterized in that the static pressure of the acoustic propagation medium contained in the first space can be set.

3. Pressure pulse generator according to Claim 1 or 2, characterized in that the quantity of the acoustic propagation medium flowing per time unit through the cooling device (20) can be set.

4. Pressure pulse generator according to one of Claims 1 to 3, characterized in that the acoustic propagation medium flows in a closed circuit through the cooling device (20).

5. Pressure pulse generator according to one of Claims 1 to 4, characterized in that the acoustic propagation medium in the first space has a flow direction directed at least essentially parallel to the surface of the diaphragm (11).

6. Pressure pulse generator according to one of Claims 1 to 5, characterized in that the wall (14) separating the first space from the second one is constructed as an acoustic lens.

7. Pressure pulse generator according to one of Claims 1 to 6, characterized in that the second space is sealed by a flexible coupling diaphragm (3) and connected to an equalization container (29).

## Revendications

1. Générateur d'impulsions de pression comprenant une source (2) d'impulsions de pression, qui comporte pour la production d'impulsions de pression acoustiques dans un milieu de propagation acoustique une membrane (11) pouvant être entraînée par à-coups, et ayant une cloison (14) opposée à la membrane (11) et séparant de manière étanche aux liquides une première chambre se trouvant entre la cloison (14) et la membrane (11) et contenant un milieu de propagation acoustique liquide d'une seconde chambre se trouvant de l'autre côté de la cloison (14) et contenant un milieu de propagation acoustique, le milieu de propagation acoustique contenu dans la première chambre étant sous une pression statique plus grande que la pression ambiante pour ramener la membrane (11) en sa position de départ et passant par un dispositif (20) de refroidissement.

2. Générateur d'impulsions de pression suivant la revendication 1, caractérisé en ce que la pression statique du milieu de propagation acoustique contenu dans la première chambre est réglable.

3. Générateur d'impulsions de pression suivant la revendication 1 ou 2, caractérisé en ce que la quantité du milieu de propagation acoustique passant par unité de temps dans le dispositif (20) de refroidissement est réglable.

4. Générateur d'impulsions de pression suivant l'une des revendications 1 à 3, caractérisé en ce que le milieu de propagation acoustique passe en circuit fermé dans le dispositif (20) de refroidissement.

5. Générateur d'impulsions de pression suivant l'une des revendications 1 à 4, caractérisé en ce que le milieu de propagation acoustique dans la première chambre a un sens d'écoulement dirigé au moins sensiblement parallèlement à la surface de la membrane (11).

6. Générateur d'impulsions de pression suivant l'une des revendications 1 à 5, caractérisé en ce que la cloison (14) séparant la première chambre de la seconde chambre est constituée en lentille acoustique.

7. Générateur d'impulsions de pression suivant l'une des revendications 1 à 6, caractérisé en ce que la seconde chambre est fermée par une membrane (3) souple de couplage et est en liaison avec un réservoir (29) de compensation.
